# EUROPEAN PATENT SPECIFICATION

(11) **EP 3 911 341 B1**
(45) Date of publication and mention of the grant of the patent: **28.12.2022**
(21) Application number: 20702576.8
(22) Date of filing: 16.01.2020
(51) Int. Cl.: A61K 35/20, A61K 31/702, A61K 35/741, A61K 35/744, A61K 35/745, A23L 33/00, A23L 33/135, A23L 33/21, A61P 1/12, A23K 50/75, A23K 20/121

(54) **FERMENTED FORMULA WITH NON-DIGESTIBLE OLIGOSACCHARIDES FOR USE IN ROTAVIRUS INDUCED INFECTION**
FERMENTIERTE FORMEL MIT UNVERDAULICHEN OLIGOSACCHARIDEN ZUR VERWENDUNG BEI ROTAVIRUS-INDUZIERTEN INFEKTIONEN
FORMULE FERMENTÉE COMPORTANT DES OLIGOSACCHARIDES NON DIGESTIBLES POUR L'UTILISATION DANS UNE INFECTION À ROTAVIRUS

(30) Priority: 16.01.2019 EP 19152161
(43) Date of publication of application: 24.11.2021
(73) Proprietor: N.V. Nutricia, 2712 HM Zoetermeer (NL)
(72) Inventor: KNIPPING, Catharina Theresia, 3584 CT Utrecht (NL); PÉREZ CANO, Francisco José, 08041 Barcelona (ES); GARSSEN, Johan, 3584 CT Utrecht (NL)
(74) Representative: Nederlandsch Octrooibureau
(86) International application number: PCT/EP2020/051034
(87) International publication number: WO 2020/148391

(56) References cited:
- KR-A- 20040 084 285
- US-A1- 2014 056 852
- RIGO-ADROVER MAR ET AL: "A fermented milk concentrate and a combination of short-chain galacto-oligosaccharides/long-chain fructo-oligosaccharides/pectin-derived acidic oligosaccharides protect suckling rats from rotavirus gastroenteritis", BRITISH JOURNAL OF NUTRITION, CAMBRIDGE UNIV. PRESS, UK, vol. 117, no. 2, 1 January 2017 (2017-01-01), pages 209-217, XP009513923, ISSN: 0007-1145, DOI: 10.1017/S0007114516004566 [retrieved on 2017-02-07]
- MARIA DEL MAR RIGO-ADROVER ET AL: "Preventive Effect of a Synbiotic Combination of Galacto- and Fructooligosaccharides Mixture With Bifidobacterium breve M-16V in a Model of Multiple Rotavirus Infections", FRONTIERS IN IMMUNOLOGY, vol. 9, 11 June 2018 (2018-06-11), XP055598092, DOI: 10.3389/fimmu.2018.01318
- MARIA RIGO-ADROVER ET AL: "Prevention of Rotavirus Diarrhea in Suckling Rats by a Specific Fermented Milk Concentrate with Prebiotic Mixture", NUTRIENTS, vol. 11, no. 1, 18 January 2019 (2019-01-18), page 189, XP055598096, DOI: 10.3390/nu11010189

## Description

### FIELD OF THE INVENTION

The present invention is in the field of nutrition for infants and young children, infant formula, for treatment or prevention of rotavirus infection

### BACKGROUND OF THE INVENTION

Viral infections are common among people of all ages but often seem to be concentrated in infants and children. Many viral infections result in fever and body aches or discomfort and are not that serious and most children with viral infections get better without treatment. Particularly during infancy, it is difficult for parents to tell whether their infant is ill with a potentially serious infection and needs immediate medical care. Rotavirus (RV) is the leading worldwide etiological agent of severe dehydrating diarrhea in childhood under the age of 5 years. RV infects and replicates mainly in the nondivid ing mature enterocytes near the tips of the small intestinal villi. As a result, infants develop diarrhea, vomiting and fever for 3 days to 1 week, usually with reinfections, which tend to be less severe than the first infection. The most common treatment consists of oral rehydration solutions (ORS). As the human RV pathogenesis is still unclear and RV vaccines are not globally implemented, the modulation by nutritional interventions with bioactive components is of interest.

Human milk is the preferred food for infants. Human milk provides several bioactive factors that benefit the relatively immature immune system of neonates early in life and may prevent or reduce the severity of rotavirus infection, such as lactoferrin and human milk oligosaccharides. However, it is not always possible or desirable to breastfeed an infant. In such cases infant formulae or follow on formulae are a good alternative. These formulae should have an optimal composition in order to mimic the beneficial effects of human milk as close as possible.

Several nutritional interventions with bioactive compounds have been studied in recent years. Among these compounds, probiotics have been the most widely studied due to their known immunomodulatory effects and direct action, often on the small intestine, which is the primary point of RV infection. WO 00/53201 discloses lactic acid bacteria strains capable of preventing diarrhea and prevent infection by rotavirus. WO 2004/2168 discloses Bifidobacteria in treatment of inflammatory diseases, including rotavirus associated diarrhea. WO 02/39834 discloses probiotic bacteria for preventing or inhibiting diarrhea rotavirus. EP 2407532 discloses that a probiotic strain and its supernatant of B. breve prevents in vitro growth of rotavirus growth in vitro. In Rigo-Adrovers et al, 2017, British Journal of Nutrition, 117, 209-217 two different concepts were tested. The first is a heat inactivated fermented formula, and the second is a specific mixture of short chain galacto-oligosaccharides, long chain fructo-oligosaccharides and pectin-derived acidic oligosaccharides. Both nutritional concepts caused amelioration of the clinical symptoms of rotavirus infection. In Rigo-Adrover et al 2018, Frontiers in Immunology, doi:10.3389/fimmu.2018.01318, a study on the preventive role of a mixture of short chain galacto-oligosaccharides and long chain fructo-oligosaccharides and of the probiotic *Bifidobacterium breve* M-16V and of the combination of the prebiotic mixture and the probiotic in a rat double-RV infection model was described. Dietary interventions ameliorated clinical symptoms after the first infection.

### SUMMARY OF THE INVENTION

The inventors have tested a combination of fermented formula and a mixture of non-digestible oligosaccharides against the effects of rotavirus infection. Surprisingly the combination of fermented formula with non-digestible oligosaccharides resulted in a much higher binding of the rotavirus particles when compared to the binding by the individual components fermented formula or non-digestible oligosaccharides. Rotavirus infection and its induced symptoms was studied using a model with young rats. Rotavirus infection induced weight loss, which was mainly observed after the diarrhea period, and rotavirus induced effects on organ weights were ameliorated in the group that received a combination of fermented formula and a mixture of non-digestible oligosaccharides. Administration of fermented formula with non-digestible oligosaccharides resulted in a reduced incidence, reduced duration and reduced severity of rotavirus induced diarrhea. In addition, a reduced virus shedding was observed. Additionally the intestinal barrier permeability in rotavirus infected animals was most decreased in the group administered the combination of fermented formula and non-digestible oligosaccharides as determined by alpha 1 antitrypsin.

### DETAILED DESCRIPTION OF THE INVENTION

The present invention thus concerns a method for treating and/or preventing rotavirus infection or rotavirus-induced diarrhea in an infant or young child, comprising administering a nutritional composition that is at least partly fermented by lactic acid producing bacteria and comprises non-digestible oligosaccharides to the infant or young child.

The invention can also be worded as the use of a fermented composition and non-digestible oligosaccharides for the manufacture of a nutritional composition that is at least partly fermented by lactic acid producing bacteria and comprises non-digestible oligosaccharides, for use in treating and/or preventing rotavirus infection or rotavirus-induced diarrhea in an infant or young child.

The invention can also be worded as a nutritional composition that is at least partly fermented by lactic acid producing bacteria and comprises non-digestible oligosaccharides, for use in treating and/or preventing rotavirus infection or rotavirus-induced diarrhea in an infant or young child.

In one embodiment, treating and/or preventing rotavirus infection or rotavirus-induced diarrhea is
a. reducing incidence of rotavirus-induced diarrhea or rotavirus infection,
b. reducing duration of rotavirus-induced diarrhea or rotavirus infection,
c. reducing severity of rotavirus-induced diarrhea or rotavirus infection,
d. preventing of rotavirus-induced reduced growth or poor growth and/or reduced development or poor development,
e. increasing blocking of rotavirus particles,
f. reducing rotaviral load, and/or
g. increasing gut barrier function during rotavirus infection.

The present invention also concerns a method for
a. reducing incidence of rotavirus-induced diarrhea or rotavirus infection,
b. reducing duration of rotavirus-induced diarrhea or rotavirus infection,
c. reducing severity of rotavirus-induced diarrhea or rotavirus infection,
d. preventing of rotavirus-induced reduced growth or poor growth and/or reduced development or poor development,
e. increasing blocking of rotavirus particles,
f. reducing rotaviral load, and/or
g. increasing gut barrier function during rotavirus infection,

in an infant or young child, comprising administering a nutritional composition that is at least partly fermented by lactic acid producing bacteria and comprises non-digestible oligosaccharides to the infant or young child.

The invention can also be worded as the use of a fermented composition and non-digestible oligosaccharides for the manufacture of a nutritional composition that is at least partly fermented by lactic acid producing bacteria and comprises non-digestible oligosaccharides, for use in
a. reducing incidence of rotavirus-induced diarrhea or rotavirus infection,
b. reducing duration of rotavirus-induced diarrhea or rotavirus infection,
c. reducing severity of rotavirus-induced diarrhea or rotavirus infection,
d. preventing of rotavirus-induced reduced growth or poor growth and/or reduced development or poor development,
e. increasing blocking of rotavirus particles,
f. reducing rotaviral load, and/or
g. increasing gut barrier function during rotavirus infection,
in an infant or young child.

The invention can also be worded as a nutritional composition that is at least partly fermented by lactic acid producing bacteria and comprises non-digestible oligosaccharides, for use in
a. reducing incidence of rotavirus-induced diarrhea or rotavirus infection,
b. reducing duration of rotavirus-induced diarrhea or rotavirus infection,
c. reducing severity of rotavirus-induced diarrhea or rotavirus infection,
d. preventing of rotavirus-induced reduced growth or poor growth and/or reduced development or poor development,
e. increasing blocking of rotavirus particles,
f. reducing rotaviral load, and/or
g. increasing gut barrier function during rotavirus infection,
in an infant or young child.

### Fermented composition

The nutritional composition in the methods or uses according to the present invention, hereafter also referred to as the present nutritional composition, or nutritional composition of the invention or final nutritional composition, is at least party fermented. A partly fermented nutritional composition comprises at least for a part a composition that was fermented by lactic acid producing bacteria. Hence, in the context of the present invention, a nutritional composition that is at least partly fermented refers to a nutritional composition that comprises a milk substrate that is fermented by lactic acid producing bacteria. It was shown that the presence of fermented composition in the final nutritional composition results, upon administration, in an effect against rotavirus diarrhea and its symptoms.

The fermentation preferably takes place during the production process of the nutritional composition. Preferably, the nutritional composition does not contain significant amounts of viable bacteria in the final product, and this can be achieved by heat inactivation after fermentation or inactivation by other means. Preferably the fermented composition is a milk-derived product, which is a milk substrate that is fermented by lactic acid producing bacteria, wherein the milk substrate comprises at least one selected from the group consisting of milk, whey, whey protein, whey protein hydrolysate, casein, casein hydrolysate or mixtures thereof. Suitably, nutritional compositions comprising fermented compositions and non-digestible oligosaccharide and their way of producing them are described in WO 2009/151330, WO 2009/151331 and WO 2013/187764.

The fermented composition preferably comprises bacterial cell fragments like glycoproteins, glycolipids, peptidoglycan, lipoteichoic acid (LTA), lipoproteins, nucleotides, and/or capsular polysaccharides. It is of advantage to use the fermented composition comprising inactivated bacteria and/or cell fragments directly as a part of the final nutritional product, since this will result in a higher concentration of bacterial cell fragments. When commercial preparations of lactic acid producing bacteria are used, these are usually washed, and material is separated from the aqueous growth medium comprising the bacterial cell fragments, thereby reducing or eliminating the presence of bacterial cell fragments. Furthermore, upon fermentation and/or other interactions of lactic acid producing bacteria with the milk substrate, additional bio-active compounds can be formed, such as short chain fatty acids, bioactive peptides and/or oligosaccharides, and other metabolites, which may also result in an intestinal microbiota-function more similar to the intestinal microbiota-function of breastfed infants. Such bioactive compounds that that are produced during fermentation by lactic acid producing bacteria may also be referred to as post-biotics. A composition comprising such post-biotics is thought to be advantageously closer to breast milk, as breast milk is not a clean synthetic formula, but contains metabolites, bacterial cells, cell fragments and the like. Therefore, the fermented composition, in particular fermented milk-derived product, is believed to have an improved effect compared to non-fermented milk-derived product without or with merely lactic acid producing bacteria on the anti-rotavirus effect.

Preferably the final nutritional composition comprises 5 to 97.5 wt.% of the fermented composition based on dry weight, more preferably 10 to 90 wt.%, more preferably 20 to 80 wt.%, even more preferably 25 to 60 wt.%. As a way to specify that the final nutritional composition comprises at least partly a fermented composition, and to specify the extent of fermentation, the level of the sum of lactic acid and lactate in the final nutritional composition can be taken, as this is the metabolic end product produced by the lactic acid producing bacteria upon fermentation. The present final nutritional composition preferably comprises 0.02 to 1.5 wt.% of the sum of lactic acid and lactate based on dry weight of the composition, more preferably 0.05 to 1.0 wt.%, even more preferably 0.2 to 0.5 wt.%. Preferably at least 50 wt.%, even more preferably at least 90 wt.%, of the sum of lactic acid and lactate is in the form of the L(+)-isomer. Thus, in one embodiment the sum of L(+)-lactic acid and L(+)-lactate is more than 50 wt.%, more preferably more than 90 wt.%, based on the sum of total lactic acid and lactate. Herein L(+)-lactate and L(+)-lactic acid is also referred to as L-lactate and L-lactic acid.

### Lactic acid producing bacteria used for producing the fermented composition

Lactic acid producing bacteria used for preparing the fermented composition, in particular for fermentation of the milk substrate are preferably provided as a mono- or mixed culture. Lactic acid producing bacteria consists of the genera *Bifidobacterium, Lactobacillus, Carnobacterium, Enterococcus, Lactococcus, Leuconostoc, Oenococcus, Pediococcus, Streptococcus, Tetragenococcus, Vagococcus and Weissella.* Preferably the lactic acid producing bacteria used for fermentation comprises bacteria of the genus *Bifidobacterium* and/or *Streptococcus.*

Preferably the *Streptococcus* is a strain of *S*. *thermophilus.* Selection of a suitable strain of *S*. *thermophilus* is described in example 2 of EP 778885 and in example 1 of FR 2723960. In a further preferred embodiment according to the present invention, the nutritional composition comprises 10²-10⁵ cfu living bacteria of S. *thermophilus,* per g dry weight of the final nutritional composition, preferably the final nutritional composition comprises 10³-10⁴ living bacteria of *S. thermophilus* per g dry weight.

Preferred strains of *S. thermophilus* to prepare the fermented composition for the purpose of the present invention have been deposited by Compagnie Gervais Danone at the Collection Nationale de Cultures de Microorganismes (CNCM) run by the Institut Pasteur, 25 rue du Docteur Roux, Paris, France on 23 August 1995 under the accession number 1-1620 and on 25 August 1994 under the accession number I-1470. Other *S. thermophilus* strains are commercially available.

*Bifidobacteria* are Gram-positive, anaerobic, rod-shaped bacteria. Preferred *Bifidobacterium* species to prepare the fermented composition for the purpose of the present invention preferably have at least 95 % identity of the 16 S rRNA sequence when compared to the type strain of the respective *Bifidobacterium* species, more preferably at least 97% identity as defined in handbooks on this subject for instance Sambrook, J., Fritsch, E.F., and Maniatis, T. (1989), Molecular Cloning, A Laboratory Manual, 2nd ed., Cold Spring Harbor (N.Y.) Laboratory Press. The Bifodobacteria preferably used are also described by Scardovi, V. Genus Bifidobacterium. p.1418- p.1434. In: Bergey's manual of systematic Bacteriology. Vol. 2. Sneath, P.H.A., N.S. Mair, M.E. Sharpe and J.G. Holt (ed.). Baltimore: Williams & Wilkins. 1986. 635 p. Preferably the lactic acid producing bacteria used for fermentation comprises or is at least one *Bifidobacterium* selected from the group consisting of *B. breve, B. infantis, B. bifidum, B. catenulatum, B. adolescentis, B. thermophilum, B. gallicum, B. animalis or lactis, B. angulatum, B. pseudocatenulatum, B. thermacidophilum* and B. *longum* more preferably *B. breve, B. infantis, B. bifidum, B. catenulatum, B. longum*, more preferably *B. longum* and *B. breve*, even more preferably *B. breve,* more preferably *B. breve* selected from the group consisting of *B. breve* Bb-03 (Rhodia/Danisco), *B. breve* M-16V (Morinaga), *B. breve* R0070 (Institute Rosell, Lallemand), *B. breve* BR03 (Probiotical), *B. breve* BR92 (Cell Biotech) DSM 20091, LMG 11613 and *B. breve* 1-2219 deposited at the CNCM, Paris France. Most preferably, the *B. breve* is *B. breve* M-16V (Morinaga) or *B. breve* I-2219, even more preferably *B. breve* I-2219.

Most preferably the nutritional composition of the invention comprises fermented composition that is fermented by lactic acid producing bacteria comprising both *B. breve* and S. *thermophilus.* In one embodiment the fermentation by lactic acid producing bacteria is fermentation by *Streptococcus thermophilus* and *Bifidobacterium breve.* In one embodiment, the final nutritional composition comprises fermented composition wherein the lactic acid producing bacteria are inactivated after fermentation.

Preferably the fermented composition is not fermented by *Lactobacillus bulgaricus. L. bulgaricus* fermented products are considered not suitable for infants, since in young infants the specific dehydrogenase that converts D-lactate to pyruvate is far less active than the dehydrogenase which converts L-lactate.

Preferably the nutritional composition of the invention comprises inactivated lactic acid producing bacteria and/or bacterial fragments derived from lactic acid producing bacteria being the equivalent of more than 1×10⁴ cfu lactic acid producing bacteria per g based on dry weight of the final composition, more preferably 1×10⁵ cfu, even more preferably 1×10⁶ cfu. Preferably the inactivated bacteria or bacterial fragments are the equivalent of less than 1×10¹³ cfu lactic acid producing bacteria per g based on dry weight of the final composition, more preferably 1x10¹¹ cfu, even more preferably 1×10¹⁰ cfu. The correlation of inactivated lactic acid bacteria and the equivalence with cfu can be determined by molecular techniques, known in the art, or by checking the production process.

### Process of fermentation

Preferably the fermented composition is a milk-derived product, which is a milk substrate that is fermented by lactic acid producing bacteria, said milk substrate comprising at least one selected from the group consisting of milk, whey, whey protein, whey protein hydrolysate, casein, casein hydrolysate or mixtures thereof. The milk derived product or milk substrate to be fermented is suitably present in an aqueous medium. The milk substrate to be fermented comprises at least one selected from the group consisting of milk, whey, whey protein, whey protein hydrolysate, casein, casein hydrolysate or mixtures thereof. Milk can be whole milk, semi-skimmed milk and/or skimmed milk. Preferably the milk substrate to be fermented comprises skimmed milk. Whey can be sweet whey, and/or acid whey. Preferably the whey is present in a concentration of 3 to 80 g dry weight per I aqueous medium containing milk substrate, more preferably 40 to 60 g per I. Preferably whey protein hydrolysate is present in 2 to 80 g dry weight per I aqueous medium containing milk substrate, more preferably 5 to15 g/l. Preferably lactose is present in 5 to 50 g dry weight per I aqueous substrate, more preferably 1 to 30 g/l. Preferably the aqueous medium containing milk substrate comprises buffer salts in order to keep the pH within a desired range. Preferably sodium or potassium dihydrogen phosphate is used as buffer salt, preferably in 0.5 to 5 g/l, more preferably 1.5 to 3 g per I. Preferably the aqueous medium containing milk substrate comprises cysteine in amount of 0.1 to 0.5 g per I aqueous substrate, more preferably 0.2 to 0.4 g/l. The presence of cysteine results in low redox potential of the substrate which is advantageous for activity of lactic acid producing bacteria, particularly *bifidobacteria.* Preferably the aqueous medium containing milk substrate comprises yeast extract in an amount of 0.5 to 5 g/l aqueous medium containing milk substrate, more preferably 1.5 to 3 g/l. Yeast extract is a rich source of enzyme co-factors and growth factors for lactic acid producing bacteria. The presence of yeast extract will enhance the fermentation by lactic acid producing bacteria.

Suitably the milk substrate, in particular the aqueous medium containing milk substrate, is pasteurised before the fermentation step, in order to eliminate the presence of unwanted living bacteria. Suitably the product is pasteurised after fermentation, in order to inactivate enzymes. Suitably the enzyme inactivation takes place at 75 °C for 3 min. Suitably the aqueous medium containing milk substrate is homogenised before and/or the milk-derived product is homogenised after the fermentation. Homogenisation results in a more stable substrate and/or fermented product, especially in the presence of fat.

The inoculation density is preferably between 1×10² to 5×10¹⁰, preferably between 1×10⁴ to 5×10⁹ cfu lactic acid producing bacteria/ml aqueous medium containing milk substrate, more preferably between 1×10⁷ to 1×10⁹ cfu lactic acid producing bacteria/ml aqueous medium containing milk substrate. The final bacteria density after fermentation is preferably between 1×10³ to 1×10¹⁰, more preferably between 1×10⁴ to 1×10⁹ cfu/ml aqueous medium containing milk substrate.

The fermentation is preferably performed at a temperature of approximately 20 °C to 50 °C, more preferably 30 °C to 45 °C, even more preferably approximately 37 °C to 42 °C. The optimum temperature for growth and/or activity for lactic acid producing bacteria, more particularly lactobacilli and/or bifidobacteria is between 37 °C and 42 °C.

The incubation is preferably performed at a pH of 4 to 8, more preferably 6 to 7.5. This pH does not induce protein precipitation and/or an adverse taste, while at the same time lactic acid producing bacteria such as lactobacilli and/or bifidobacteria are able to ferment the milk substrate.

The incubation time preferably ranges from 10 minutes to 48 h, preferably from 2 h to 24 h, more preferably from 4 h to 12 h. A sufficient long time enables fermentation and the concomitant production of immunogenic cell fragments such as glycoproteins, glycolipids, peptidoglycan, lipoteichoic acid (LTA), flagellae, lipoproteins, DNA and/or capsular polysaccharides and metabolites (postbiotics) to take place at a sufficient or higher extent, whereas the incubation time needs not be unnecessarily long for economic reasons.

Preferably, a milk derived product or milk substrate, preferably skimmed milk, is pasteurized, cooled and fermented with one or more lactic acid producing strains, preferably a strain of *S. thermophilus,* to a certain degree of acidity, upon which the fermented product is cooled and stored. Preferably a second milk-derived product is prepared in a similar way using one or more *Bifidobacterium* species for fermentation. Subsequently, the two fermented products are preferably mixed together and mixed with other components making up an infant formula, except the fat component. Preferably, the mixture is preheated, and subsequently fat is added in-line, homogenized, pasteurized and dried. Alternatively, the fermentation takes place having both *Bifidobacterium,* preferably *B. breve,* and *S. thermophilus* in the fermentation tank.

Procedures to prepare fermented composition suitable for the purpose of the present invention are known per se. EP 778885, which is incorporated herein by reference, discloses in particular in example 7 a suitable process for preparing a fermented composition. FR 2723960, which is incorporated herein by reference, discloses in particular in example 6 a suitable process for preparing a fermented composition. Briefly, a milk substrate, preferably pasteurised, containing lactose and optionally further macronutrients such as fats, preferably vegetable fats, casein, whey protein, vitamins and/or minerals etc. is concentrated, e.g. to between 15 to 50% dry matter and then inoculated with *S. thermophilus,* for example with 5% of a culture containing 10⁶ to 10¹⁰ bacteria per ml. Preferably this milk substrate comprises milk protein peptides. Temperature and duration of fermentation are as mentioned above. Suitably after fermentation the fermented composition may be pasteurised or sterilized and for example spray dried or lyophilised to provide a form suitable to be formulated in the end product.

A preferred method for preparing the fermented composition to be used in the nutritional composition of invention is disclosed in WO 01/01785, more particular in examples 1 and 2. A preferred method for preparing the fermented composition to be used in the nutritional composition of invention is described in WO 2004/093899, more particularly in example 1.

Living cells of lactic acid producing bacteria in the fermented composition are after fermentation preferably eliminated, for example by inactivation and/or physical removal. The cells are preferably inactivated. Preferably the lactic acid producing bacteria are heat killed after fermentation of the milk substrate. Preferable ways of heat killing are (flash) pasteurization, sterilization, ultra-high temperature treatment, high temperature/short time heat treatment, and/or spray drying at temperatures bacteria do not survive. Cell fragments are preferably obtained by heat treatment. With this heat treatment preferably at least 90 % of living microorganisms are inactivated, more preferably at least 95 %, even more preferably at least 99 %. Preferably the fermented nutritional composition comprises less than 1×10⁵ colony forming units (cfu) living lactic acid bacteria per g dry weight. The heat treatment preferably is performed at a temperature ranging from 70 to180 °C, preferably from 80 to 150 °C, preferably for about 3 minutes to 2 hours, preferably in the range of 80 to 140 °C for 5 minutes to 40 minutes. Inactivation of the lactic acid bacteria advantageously results in less post acidification and a safer product. This is especially advantageous when the nutritional composition is to be administered to infants ortoddlers and yet still with a fermented composition comprising inactivated lactic acid bacteria beneficial effects on rotavirus infections were observed. Suitably after fermentation the fermented composition may be pasteurised or sterilized and for example spray dried or lyophilised to provide a form suitable to be formulated in the end product.

### Non-digestible oligosaccharides

The present nutritional composition comprises non-digestible oligosaccharides and preferably comprises at least two different non-digestible oligosaccharides, in particular two different sources of non-digestible oligosaccharides. The presence of non-digestible oligosaccharides is needed to improve the effects on rotavirus infection. The presence of both the non-digestible oligosaccharides and the at least partly fermented composition, in particular the milk-derived product obtained by fermentation with lactic acid producing bacteria, is needed to improve an anti-rotavirus effect, in particular on the binding of the rotavirus particles, or the blocking of particles from adhesion sites on the intestinal epithelial cells.

The term "oligosaccharides" as used herein refers to saccharides with a degree of polymerization (DP) of 2 to 250, preferably a DP 2 to 100, more preferably 2 to 60, even more preferably 2 to 10. If oligosaccharide with a DP of 2 to 100 is included in the present nutritional composition, this results in compositions that may contain oligosaccharides with a DP of 2 to 5, a DP of 50 to 70 and a DP of 7 to 60. The term "non-digestible oligosaccharides" as used in the present invention refers to oligosaccharides which are not digested in the intestine by the action of acids or digestive enzymes present in the human upper digestive tract, e.g. small intestine and stomach, but which are preferably fermented by the human intestinal microbiota. For example, sucrose, lactose, maltose and maltodextrins are considered digestible.

Preferably the present non-digestible oligosaccharides are soluble. The term "soluble" as used herein, when having reference to a polysaccharides, fibres or oligosaccharides, means that the substance is at least soluble according to the method described by L. Prosky et al., J. Assoc. Off. Anal. Chem. 71, 1017-1023 (1988).

The non-digestible oligosaccharides included in the present nutritional compositions in the methods or uses according to the present invention preferably include a mixture of different non-digestible oligosaccharides. The non-digestible oligosaccharides are preferably selected from the group consisting of fructo-oligosaccharides, such as inulin, non-digestible dextrins, galacto-oligosaccharides, such as transgalacto-oligosaccharides, xylo-oligosaccharides, arabino-oligosaccharides, arabinogalacto-oligosaccharides, gluco-oligosaccharides, gentio-oligosaccharides, glucomanno-oligosaccharides, galactomanno-oligosaccharides, mannan-oligosaccharides, isomalto-oligosaccharides, nigero-oligosaccharides, glucomanno-oligosaccharides, chito-oligosaccharides, soy oligosaccharides, fuco-oligosaccharides, sialyloligosaccharides and mixtures thereof. Such oligosaccharides share many biochemical properties and have similar functional benefits including improving the intestinal microbiota-function. Yet is understood that some non-digestible oligosaccharides and preferably some mixtures have an even further improved effect. Therefore, more preferably the non-digestible oligosaccharides are selected from the group consisting of fructo-oligosaccharides, such as inulin, and galacto-oligosaccharides, such as betagalacto-oligosaccharides, and mixtures thereof, even more preferably betagalacto-oligosaccharides and/or inulin, most preferably betagalacto-oligosaccharides. In one embodiment in the nutritional composition according to the present invention, the non-digestible oligosaccharides are selected from the group consisting of galacto-oligosaccharides, fructo-oligosaccharides and mixtures of thereof, more preferably betagalacto-oligosaccharides, fructo-oligosaccharides and mixtures thereof.

The non-digestible oligosaccharide is preferably selected from the group consisting of beta-galacto-oligosaccharide, alpha-galacto-oligosaccharide, and galactan. According to a more preferred embodiment non-digestible oligosaccharide is beta-galacto-oligosaccharide. Preferably the non-digestible oligosaccharide comprises galacto-oligosaccharide with beta(1,4), beta(1,3) and/or beta(1,6) glycosidic bonds and a terminal glucose. Transgalacto-oligosaccharide is for example available under the trade name Vivinal^{®}GOS (Domo FrieslandCampina Ingredients), Bi2muno (Clasado), Cup-oligo (Nissin Sugar) and Oligomate55 (Yakult).

The non-digestible oligosaccharides preferably comprise fructo-oligosaccharides. Fructo-oligosaccharides may in other context have names like fructopolysaccharides, oligofructose, polyfructose, polyfructan, inulin, levan and fructan and may refer to oligosaccharides comprising beta-linked fructose units, which are preferably linked by beta(2,1) and/or beta(2,6) glycosidic linkages, and a preferable DP between 2 and 200. Preferably, the fructo-oligosaccharides contain a terminal beta(2,1) glycosidic linked glucose. Preferably, the fructo-oligosaccharides contain at least 7 beta-linked fructose units. In a further preferred embodiment inulin is used. Inulin is a type of fructo-oligosaccharide wherein at least 75% of the glycosidic linkages are beta(2,1) linkages. Typically, inulin has an average chain length between 8 and 60 monosaccharide units. A suitable fructo-oligosaccharide for use in the compositions of the present invention is commercially available under the trade name Raftiline^{®}HP (Orafti). Other suitable sources are Raftilose (Orafti), Fibrulose and Fibruline (Cosucra) and Frutafit and Frutalose (Sensus).

Preferably the present nutritional composition comprises a mixture of galacto-oligosaccharides and fructo-oligosaccharides. Preferably the mixture of galacto-oligosaccharides and fructo-oligosaccharides is present in a weight ratio of from 1/99 to 99/1, more preferably from 1/19 to 19/1, more preferably from 1/1 to 19/1, more preferably from 2/1 to 15/1, more preferably from 5/1 to 12/1, even more preferably from 8/1 to 10/1, even more preferably in a ratio of about 9/1. This weight ratio is particularly advantageous when the galacto-oligosaccharides have a low average DP and fructo-oligosaccharides has a relatively high DP. Most preferred is a mixture of galacto-oligosaccharides with an average DP below 10, preferably below 6, and fructo-oligosaccharides with an average DP above 7, preferably above 11, even more preferably above 20.

Preferably the present nutritional composition comprises a mixture of short chain fructo-oligosaccharides and long chain fructo-oligosaccharides. Preferably the mixture of short chain fructo-oligosaccharides and long chain fructo-oligosaccharides is present in a weight ratio of from 1/99 to 99/1, more preferably from 1/19 to 19/1, even more preferably from 1/10 to 19/1, more preferably from 1/5 to 15/1, more preferably from 1/1 to 10/1. Preferred is a mixture of short chain fructo-oligosaccharides with an average DP below 10, preferably below 6 and a fructo-oligosaccharides with an average DP above 7, preferably above 11, even more preferably above 20.

Preferably the present nutritional composition comprises a mixture of short chain fructo-oligosaccharides and short chain galacto-oligosaccharides. Preferably the mixture of short chain fructo-oligosaccharides and short chain galacto-oligosaccharides is present in a weight ratio of from 1/99 to 99/1, more preferably from 1/19 to 19/1, even more preferably from 1/10 to 19/1, more preferably from 1/5 to 15/1, more preferably from 1/1 to 10/1. Preferred is a mixture of short chain fructo-oligosaccharides and galacto-oligosaccharides with an average DP below 10, preferably below 6.

Preferably the nutritional composition does not comprise pectin-derived acidic oligosaccharides. Such oligosaccharides are rich in galacturonic acid oligosaccharide. In the context of the present invention, pectin-derived acidic oligosaccharides are characterized as being a mixture of oligomers of galacturonic acid with a degree of polymerization of 2-30. Therefore preferably the present composition does not comprise galacturonic acid oligosaccharides. These oligosaccharides are well known for their ability to block adhesion of pathogens to receptors in the intestine. Yet surprisingly its presence was not needed to obtain a maximal blocking effect of rotavirus particles, when non-digestible oligosaccharides were combined with a fermented nutritional composition.

The present nutritional composition preferably comprises 2.5 to 20 wt.% total non-digestible oligosaccharides, more preferably 2.5 to 15 wt.%, even more preferably 3.0 to 10 wt.%, most preferably 5.0 to 7.5 wt.%, based on dry weight of the nutritional composition. Based on 100 ml the present nutritional composition preferably comprises 0.35 to 2.5 wt.% total non-digestible oligosaccharides, more preferably 0.35 to 2.0 wt.%, even more preferably 0.4 to 1.5 wt.%, based on 100 ml of the nutritional composition. A lower amount of non-digestible oligosaccharides will be less effective in improving the anti-rotavirus effects, whereas a too high amount will result in side-effects of bloating and abdominal discomfort.

### Nutritional composition

The nutritional composition used according to the present invention may also be considered as being a pharmaceutical composition and is suitable for administration to infants. The present nutritional composition is preferably for enteral administration, more preferably for oral administration.

The present nutritional composition is preferably an infant formula, follow on formula, toddler milk or toddler formula, or growing up milk intended for young children or young child formula. More preferably the nutritional composition is an infant formula or follow on formula. The present nutritional composition can be advantageously applied as a complete nutrition for infants. Preferably the present nutritional composition is an infant formula. An infant formula is defined as a formula for use in infants and can for example be a starter formula, intended for infants of 0 to 6 or 0 to 4 months of age. A follow-on formula is intended for infants of 4 or 6 months to 12 months of age. At this age infants start weaning on other food. A toddler or growing up milk or young child formula is intended for children of 12 to 36 months of age. The present composition preferably comprises a lipid component, protein component and carbohydrate component and is preferably administered in liquid form. The present nutritional composition may also be in the form of a dry food, preferably in the form of a powder which is accompanied with instructions as to mix said dry food, preferably powder, with a suitable liquid, preferably water. The nutritional composition used according to the invention preferably comprises other fractions, such as vitamins, minerals, trace elements and other micronutrients in order to make it a complete nutritional composition. Preferably infant formulae comprise vitamins, minerals, trace elements and other micronutrients according to international directives.

The present nutritional composition preferably comprises lipid, protein and digestible carbohydrate wherein the lipid provides 5 to 50% of the total calories, the protein provides 5 to 50% of the total calories, and the digestible carbohydrate provides 15 to 90% of the total calories. Preferably, in the present nutritional composition the lipid provides 35 to 50% of the total calories, the protein provides 7.0 to 12.5% of the total calories, and the digestible carbohydrate provides 40 to 55% of the total calories. For calculation of the % of total calories for the protein, the total of energy provided by proteins, peptides and amino acids needs to be taken into account. Preferably the lipid provides 3 to 7 g lipid per 100 kcal, preferably 4 to 6 g per 100 kcal, the protein provides 1.6 to 4 g per 100 kcal, preferably 1.7 to 2.5 g per 100 kcal and the digestible carbohydrate provides 5 to 20 g per 100 kcal, preferably 8 to 15 g per 100 kcal of the nutritional composition. Preferably the present nutritional composition comprises lipid providing 4 to 6 g per 100 kcal, protein providing 1.6 to 2.0 g per 100 kcal, more preferably 1.7 to 1.9 g per 100 kcal and digestible carbohydrate providing 8 to 15 g per 100 kcal of the nutritional composition. In one embodiment, the lipid provides 3 to 7 g lipid per 100 kcal, preferably 4 to 6 g per 100 kcal, the protein provides 1.6 to 2.1 g per 100 kcal, preferably 1.6 to 2.0 g per 100 kcal and the digestible carbohydrate provides 5 to 20 g per 100 kcal, preferably 8 to 15 g per 100 kcal of the nutritional composition and wherein preferably the digestible carbohydrate component comprises at least 60 wt.% lactose based on total digestible carbohydrate, more preferably at least 75 wt.%, even more preferably at least 90 wt.% lactose based on total digestible carbohydrate. The amount of total calories is determined by the sum of calories derived from protein, lipids, digestible carbohydrates and non-digestible oligosaccharide.

The present nutritional composition preferably comprises a digestible carbohydrate component. Preferred digestible carbohydrate components are lactose, glucose, sucrose, fructose, galactose, maltose, starch and maltodextrin. Lactose is the main digestible carbohydrate present in human milk. The present nutritional composition preferably comprises lactose. As the present nutritional composition comprises a fermented composition that is obtained by fermentation by lactic acid producing bacteria, the amount of lactose is reduced compared to its source due to the fermentation whereby lactose is converted into lactate and/or lactic acid. Therefore, in the preparation of the present nutritional composition lactose is preferably added. Preferably the present nutritional composition does not comprise high amounts of carbohydrates other than lactose. Compared to digestible carbohydrates such as maltodextrin, sucrose, glucose, maltose and other digestible carbohydrates with a high glycemic index, lactose has a lower glycemic index and is therefore preferred. The present nutritional composition preferably comprises digestible carbohydrate, wherein at least 35 wt.%, more preferably at least 50 wt.%, more preferably at least 60 wt.%, more preferably at least 75 wt.%, even more preferably at least 90 wt.%, most preferably at least 95 wt.% of the digestible carbohydrate is lactose. Based on dry weight the present nutritional composition preferably comprises at least 25 wt.% lactose, preferably at least 40 wt.%, more preferably at least 50 wt.% lactose.

The present nutritional composition preferably comprises at least one lipid selected from the group consisting of animal lipid (excluding human lipids) and vegetable lipids. Preferably the present composition comprises a combination of vegetable lipids and at least one oil selected from the group consisting of fish oil, animal oil, algae oil, fungal oil, and bacterial oil. The lipid of the present nutritional composition preferably provides 3 to 7 g per 100 kcal of the nutritional composition, preferably the lipid provides 4 to 6 g per 100 kcal. When in liquid form, e.g. as a ready-to-feed liquid, the nutritional composition preferably comprises 2.1 to 6.5 g lipid per 100 ml, more preferably 3.0 to 4.0 g per 100 ml. Based on dry weight the present nutritional composition preferably comprises 12.5 to 40 wt.% lipid, more preferably 19 to 30 wt.%. Preferably the lipid comprises the essential fatty acids alpha-linolenic acid (ALA), linoleic acid (LA) and/or long chain polyunsaturated fatty acids (LC-PUFA). The LC-PUFA, LA and/or ALA may be provided as free fatty acids, in triglyceride form, in diglyceride form, in monoglyceride form, in phospholipid form, or as a mixture of one of more of the above. Preferably the present nutritional composition comprises at least one, preferably at least two lipid sources selected from the group consisting of rape seed oil (such as colza oil, low erucic acid rape seed oil and canola oil), high oleic sunflower oil, high oleic safflower oil, olive oil, marine oils, microbial oils, coconut oil, palm kernel oil. The present nutritional composition is not human milk.

The present nutritional composition preferably comprises protein. The protein used in the nutritional composition is preferably selected from the group consisting of non-human animal proteins, preferably milk proteins, vegetable proteins, such as preferably soy protein and/or rice protein, and mixtures thereof. The present nutritional composition preferably contains casein, and/or whey protein, more preferably bovine whey proteins and/or bovine casein. Thus, in one embodiment the protein in the present nutritional composition comprises protein selected from the group consisting of whey protein and casein, preferably whey protein and casein, preferably the whey protein and/or casein is from cow's milk. Preferably the protein comprises less than 5 wt.% based on total protein of free amino acids, dipeptides, tripeptides or hydrolysed protein. The present nutritional composition preferably comprises casein and whey proteins in a weight ratio casein : whey protein of 10 : 90 to 90 : 10, more preferably 20 : 80 to 80 : 20, even more preferably 35 : 65 to 55 : 45.

The wt.% protein based on dry weight of the present nutritional composition is calculated according to the Kjeldahl-method by measuring total nitrogen and using a conversion factor of 6.38 in case of casein, or a conversion factor of 6.25 for other proteins than casein. The term 'protein' or 'protein component' as used in the present invention refers to the sum of proteins, peptides and free amino acids.

The present nutritional composition preferably comprises protein providing 1.6 to 4.0 g protein per 100 kcal of the nutritional composition, preferably providing 1.6 to 3.5 g, even more preferably 1.75 to 2.5 g per 100 kcal of the nutritional composition. In one embodiment, the present nutritional composition comprises protein providing 1.6 to 2.1 g protein per 100 kcal of the nutritional composition, preferably providing 1.6 to 2.0 g, more preferably 1.7 to 2.1 g, even more preferably 1.75 to 2.0 g per 100 kcal of the nutritional composition. In one embodiment, the present nutritional composition comprises protein in an amount of less than 2.0 g per 100 kcal, preferably providing 1.6 to 1.9 g, even more preferably 1.75 to 1.85 g per 100 kcal of the nutritional composition. A too low protein content based on total calories will result is less adequate growth and development in infants and young children. A too high amount will put a metabolic burden, e.g. on the kidneys of infants and young children. When in liquid form, e.g. as a ready-to-feed liquid, the nutritional composition preferably comprises 0.5 to 6.0 g, more preferably 1.0 to 3.0 g, even more preferably 1.0 to 1.5 g protein per 100 ml, most preferably 1.0 to 1.3 g protein per 100 ml. Based on dry weight the present nutritional composition preferably comprises 5 to 20 wt.% protein, preferably at least 8 wt.% protein based on dry weight of the total nutritional composition, more preferably 8 to 14 wt.%, even more preferably 8 to 9.5 wt.% protein based on dry weight of the total nutritional composition.

In order to meet the caloric requirements of an infant or toddler, the nutritional composition preferably comprises 45 to 200 kcal/100 ml liquid. For infants the nutritional composition has more preferably 60 to 90 kcal/100 ml liquid, even more preferably 65 to 75 kcal/100 ml liquid. This caloric density ensures an optimal ratio between water and calorie consumption. For toddlers, human subjects with an age between 12 and 36 months, the nutritional composition more preferably has a caloric density between 45 and 65, even more preferably between 50 and 60 kcal/100 ml. The osmolarity of the present composition is preferably between 150 and 420 mOsmol/l, more preferably 260 to 320 mOsmol/l. The low osmolarity aims to further reduce the gastrointestinal stress, which may affect rotavirus infection.

When the nutritional composition is in a ready to feed, liquid form, the preferred volume administered on a daily basis is in the range of about 80 to 2500 ml, more preferably about 200 to 1200 ml per day. Preferably, the number of feedings per day is between 1 and 10, preferably between 3 and 8. In one embodiment the nutritional composition is administered daily for a period of at least 2 days, preferably for a period of at least 4 weeks, preferably for a period of at least 8 weeks, more preferably for a period of at 25 least 12 weeks, in a liquid form wherein the total volume administered daily is between 200 ml and 1200 ml and wherein the number of feedings per day is between 1 and 10.

The present nutritional composition, when in liquid form, preferably has a viscosity between 1 and 60 mPa.s, preferably between 1 and 20 mPa.s, more preferably between 1 and 10 mPa.s, most preferably between 1 and 6 mPa.s. The low viscosity ensures a proper administration of the liquid, e.g. a proper passage through the whole of a nipple. Also, this viscosity closely resembles the viscosity of human milk. Furthermore, a low viscosity results in a normal gastric emptying and a better energy intake, which is essential for infants which need the energy for optimal growth and development. The present nutritional composition alternatively is in powder form, suitable for reconstitution with water to a ready to drink liquid. The present nutritional composition is preferably prepared by admixing a powdered composition with water. Normally infant formula is prepared in such a way. The present invention thus also relates to a packaged power composition wherein said package is provided with instructions to admix the powder with a suitable amount of liquid, thereby resulting in a liquid composition with a viscosity between 1 and 60 mPa.s. The viscosity of the liquid is determined using a Physica Rheometer MCR 300 (Physica Messtechnik GmbH, Ostfilden, Germany) at a shear rate of 95 s⁻¹ at 20 °C.

### Application

The methods or uses according to the present invention comprising administering the present nutritional composition also refer to administering an effective amount of the nutritional composition to a subject in in need of such treatment, preferably an infant or young child.

The inventors found that a combination of fermented formula (providing postbiotic compounds) and a mixture of non-digestible oligosaccharides showed the capacity to ameliorate rotavirus infection and rotavirus induced diarrhea. The non-digestible oligosaccharide enriched fermented formula reduced the incidence, duration and severity of the diarrhea process. In addition, the combination was able to directly interact with the virus and to enhance the host immune system. An unexpected improved effect of the fermented formula with non-digestible oligosaccharides was observed when compared to fermented formula not comprising non-digestible oligosaccharides or non-digestible oligosaccharides, when assaying direct blocking of the rotavirus particles. This is indicative of reducing the rotaviral load in the gastrointestinal tract. Viral load, also known as viral burden, or viral titer, is a numerical expression of the quantity of infectious viral particles per ml. A higher viral burden, titer or load often correlates with the severity or infection.

Additionally, the largest anti-rotavirus effects on duration, incidence and severity were observed in the group which was administered both non-digestible oligosaccharides and fermented formula. Especially the effect on a shortened duration was observed only in this group and cannot be explained by the effects of the non-digestible oligosaccharides or fermented formula alone. Interestingly, the combination of fermented formula with non-digestible oligosaccharides increased the gut barrier function during rotavirus infection to the largest extent, as assessed by intestinal barrier permeability using the alpha 1 antitrypsin assay. Without wishing to be bound by theory, the observed improvement in intestinal barrier function may be explained by the unexpected increase in intestinal butyric acid. Butyrate is known as a fuel for the intestinal epithelial cells and to improve the gut barrier. The increased level of butyric acid is more than can be expected based on the levels found for group fed non-digestible oligosaccharides or fermented formula alone.

Without wishing to be bound by theory, the lower viral shedding following the intervention suggests that the components assayed may have an effect on binding with the virus. This microbiota-independent anti-infective effect of the combination of non-digestible oligosaccharides and fermented formula may involve a direct interaction with pathogens and may be associated with receptor mimicry. Thus, some components of the mixture administered may be acting as soluble receptor analogues for specific molecules of the host to which the virus could adhere. Therefore, this interaction between the pathogen and the combination of fermented formula and non-digestible oligosaccharides may dislodge the adherent pathogen from the epithelial cells in the small intestine, thereby reducing the infection load or, the infectivity of the rotavirus and thus reducing the incidence, duration and/or severity of diarrhea. In addition, a reduced virus shedding and a different systemic and intestinal immune response was observed. Also, of importance is the finding that rotavirus infection induced a reduced weight gain, mainly observed after the diarrhea period, and this rotavirus infection induced effects on weight gain were ameliorated in the group that received a combination of fermented formula and mixture of non-digestible oligosaccharides. Not only effects on growth, in particular body weight, were observed, but also effects on the relative weights of organs were observed in the group that was infected with rotavirus. The spleen, liver, small intestine and large intestine were reduced in weight, even in relative weight (weight % of total body weight) the RV group when compared to the reference group. This is indicative that not only growth but also the development is affected after a rotavirus infection or rotavirus-induced diarrhea. However, these effects were ameliorated, and more similar to the non-infected reference group, in the group that received a combination of fermented formula and mixture of non-digestible oligosaccharides. Prevention of weight loss or prevention of a reduction in growth or poor growth (in particular weight gain), or prevention of reduced development of organs like liver, spleen, small intestine and/or large intestine is of is of utmost importance in the developing and growing infant or young child. These observations are indicative of both an improved growth (in particular weight gain) and an improved development of the infant or young child after a rotavirus infection or rotavirus induced diarrhea, when the nutritional composition of the present invention is administered.

Therefore, in one embodiment the present invention relates to a method or use for preventing rotavirus infection-induced reduced growth or poor growth and/or reduced development or poor reduced, more preferably reduced weight gain or poor weight gain, in infants or young children. Preferably the method or use for preventing rotavirus infection-induced reduced growth or poor growth and/or reduced development or poor development in infants or young children is the prevention of reduced growth or poor growth and/or reduced development or poor development in the period after the rotavirus infection or rotavirus-induced diarrhea. This method or use can be achieved by administering a nutritional composition that is at least partly fermented by lactic acid producing bacteria and comprises non-digestible oligosaccharides.

Therefore, in one embodiment the current invention relates to a method or use for preventing and/or treating rotavirus infection or rotavirus-induced diarrhea. More in particular in different embodiments, the effect on rotavirus infection or rotavirus-induced diarrhea is a reduction in incidence of rotavirus-induced diarrhea or rotavirus infection, or a reduction in duration of rotavirus-induced diarrhea or rotavirus infection, or a reduction in severity of rotavirus-induced diarrhea or rotavirus infection. In further embodiments, the effect on rotavirus infection or rotavirus-induced diarrhea is prevention of rotavirus-induced reduced growth or poor growth and/or reduced development or poor development, or increasing blocking of rotavirus particles, or reducing rotaviral load or increasing gut barrier function during rotavirus infection. The invention in one embodiment relates to a method or use for blocking of rotavirus particles, reducing rotaviral load and/or reducing rotavirus shedding. This is achieved by administering a nutritional composition as described above, that is at least partly fermented by lactic acid producing bacteria and comprises non-digestible oligosaccharides, in particular scGOS and IcFOS, to an in an infant or young child.

In further embodiments, the current invention relates to a method for reducing incidence of rotavirus-induced diarrhea or rotavirus infection, or reducing duration of rotavirus-induced diarrhea or rotavirus infection, or reducing severity of rotavirus-induced diarrhea or rotavirus infection, or preventing of rotavirus-induced reduced growth or poor growth and/or reduced development or poor development, or increasing blocking of rotavirus particles, or reducing rotaviral load or increasing gut barrier function during rotavirus infection, by administering a nutritional composition as described above, that is at least partly fermented by lactic acid producing bacteria and comprises non-digestible oligosaccharides, in particular scGOS and IcFOS, to an in an infant or young child.

For all the methods and uses the claimed advantageous effects are when compared to infants not being administered the nutritional composition of the present of invention; in other words, when compared to infants being administered a nutritional composition that is not at least partly fermented and does not comprise non-digestible oligosaccharides.

The present nutritional composition is administered to an infant or young child, with an age of 0 to 36 month. Preferably the nutritional composition is administered to an infant, i.e. a human subject with an age of 0 to 12 months, more preferably in an infant with an age of 0 to 6 months.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one. Wt.% means weight percentage. Unless mentioned otherwise a day refers to a period of 24 h (starting and ending at midnight).

### EXAMPLES

### Example 1: Effect of fermented formula with non-digestible oligosaccharides on rotavirus infection in rats. Materials and Methods

The rotavirus (RV) strain selected for the infection (simian SA-11) was produced described in Perez-Cano F J, et al, 2007, Pediatr. Res. 2007;62: 658-663. Briefly, propagation of viruses was done in fetal African green monkey kidney cells (MA-104) and titrated as TCID₅₀/mL (TCID, tissue culture infectious dose). Viruses were produced in compliance with the current principles of Good Laboratory Practices.

G14 pregnant Lewis rats were obtained from Janvier (Le-Genest-Saint-Isle, France) and housed individually in cages (2184L Eurostandard Type II L, Tecniplast, West Chester, PA, USA) with large fibrous particle bedding and tissue paper as enrichment. They were monitored daily and allowed to deliver at term. The day of birth was registered as day 1 of life. Litters were unified to seven pups/dam with a similar number of each sex in each nest. The pups had free access to the nipples and the rat diet. Dams had access to water and the commercial diet (Teklad Global Diet 2014, Envigo, Indianapolis, IN, USA) corresponding to the American Institute of Nutrition (AIN)-93G diet ad libitum. Each dam and its litter were housed individually under controlled temperature and humidity conditions, in a 12 h:12 h light:dark cycle. Furthermore, the animals were located in a special safe isolated room, specially designed and authorized for working under biosecurity level 2 conditions.

Experimental nutrition or nutritional components tested were:
1) A heat treated fermented infant formula. The fermented milk was obtained by fermentation of an infant formula with *Bifidobacterium breve* C50 and *Streptococcus thermophilus* 065 during the manufacturing process. This process added to the composition of the formula active bacterial metabolites and cell fragments (postbiotic compounds). It has been available on the market in France under the name Calisma and Apaisia, as a product from Bledina. Suitable methods on how to obtain this infant formula is described in example 1 of WO 2004/093899.
2) A mixture of non-digestible oligosaccharides scGOS (source Vivinal^{®} GOS, Borculo Domo FrieslandCampina) and IcFOS (Source Raftiline^{®} HP, Orafti) in a 9:1 wt./wt. ratio
3) Vehicle: The vehicle of the nutritional composition or ingredients in 1 and 2 was mineral water.

Newborn, suckling G14 Lewis rats were distributed into several different experimental groups:
1) reference (REF) group, that was a control group that was not infected with rotavirus, and received no dietary intervention
2) a rotavirus (RV) group), that was a control group that was infected with rotavirus and received no dietary intervention
3) a study group (FM+G/F), that was infected with rotavirus and that was supplemented with fermented milk (FM) as described above under and a mixture of non-digestible oligosaccharides scGOS/IcFOS (G/F). FM and G/F were administered in an amount of 3 g/100 g body weight/day and 0.8 g/100 g body weight/day, respectively.

Each group was composed of three litters with seven pups each (n=21/group). The REF and RV group received vehicle in the same amount as the FM+G/F group. Diets or vehicle were administered from day 3 of life to the last day of the experiment. The RV inoculation was carried out at day 7 of life.

SA-11 was inoculated (2×10⁸ TCID₅₀/rat in 100 µL of phosphate-buffered solution (PBS), at day 7 of life in suckling rats from the RV and FM+G/F group. The RV inoculations were performed 1 h after separation from their dams to avoid interference between RV and milk components. The REF group received the same volume of PBS under the same conditions.

SA-11 infection was evaluated on days 8-21 by the growth rate and clinical indexes derived from fecal samples. Fecal sampling was performed once a day by gently pressing and massaging the abdomen. Specimens were immediately scored, weighed, and frozen at -20 °C for further analysis.

Animals were euthanized at two different time points: three animals of each litter were euthanized 1 week after the RV inoculation, on day 14 (n=9/group), while the other four animals of each litter continued receiving the diet until the end of the suckling period, on day 21 (n=12/group). The day of the sacrifice, blood was collected by cardiac puncture for hematocrit (HCT) determination and sera obtainment, which was stored at -20 °C until further analysis. Liver, spleen, large intestine and small intestine were weighed. In addition, the small intestine was cut into 5 mm pieces and incubated with PBS for 10 min at 37 °C in a shaker to obtain the gut wash (GW). After centrifugation, supernatants were stored at -20 °C until analysis.

The severity of diarrhea was evaluated by both the fecal weight and by scoring stools. Fecal specimens were scored from 1 to 4 (diarrhea index, DI) based on color, texture and amount according to the following scale: normal (diarrhea index=1), loose yellow-green (diarrhea index=2), totally loose yellow-green (diarrhea index=3), high amount of watery (diarrhea index=4) feces. A diarrhea score ≥ 2 indicates diarrheic feces whereas scores of diarrhea index < 2 indicate absence of diarrhea. In addition, in order to find a global value of severity of diarrhea the analysis of the area under the curve (AUC) of the severity during the diarrhea period (days 7-13) was performed. The maximum diarrhea index was defined as the highest score during the diarrhea period.

Incidence of diarrhea was expressed by the % of diarrheic animals (%DA), considering the number of animals in each group, and by the % of diarrheic feces (%DF), taking into account the number of total samples collected every day in each group. The AUCs of the % of diarrheic animals and the % of diarrheic feces during days 7-13 were calculated as a global value of incidence. The maximum % of diarrheic animals and diarrheic feces were defined as the highest values during the diarrhea period. The diarrhea period was calculated for each animal as the interval between the first and the last day of diarrhea. The actual days with diarrhea within the diarrhea period were also counted.

In all cases, the mean values and AUCs for severity, % DA and % DF were calculated considering the basal values due to intrinsic aspects of each treatment; in essence subtracting the basal values of the day before inoculation with the rotavirus due to intrinsic aspects of each treatment (normalized results). This effect is in particular prominent in the groups consuming G/F due to an intrinsic stool softening effect.

UV-inactivated SA-11 particles at 10⁵/mL were used for coating 96-well plates (Nunc MaxiSorp, Wiesbaden, Germany). After blocking with PBS-1% bovine serum albumin (BSA, 1 h, room temperature [RT]), appropriate diluted sera or intestinal wash samples were added (3 h, RT). Rabbit anti-rat Ig conjugated to peroxidase from Dako (Barcelona, Spain) or mouse biotinylated anti-rat IgA (A93-2), IgG1/2a (R19-15) or IgM (G53-238) monoclonal antibodies (MAb) from BD Biosciences (Heidelberg, Germany) were added after washing. Afterwards, peroxidase-conjugated extravidin (Sigma-Aldrich, Madrid, Spain) and substrate were added. Pooled sera from RV-infected animals from previous studies were used as control positive in each plate. Quadratic polynomial adjustment was used. The concentration of total anti-RV Ab and anti-RV IgG, IgA and IgM Abs from the RV group received the value of 100 arbitrary units (AU)/mL and the results of the FM+P group and those from day 21 were expressed with respect to this given value.

Fecal samples from up to 10 days after virus inoculation (7-16 days of age) were diluted in PBS at 20 mg/mL and homogenized using a Polytron (Kinematica, Luzern, Switzerland). Fecal homogenates were centrifuged (200 g, 5 min, 4 °C), and supernatants were frozen at -20 °C until use. Viral particles in fecal homogenates were quantified by the above-mentioned ELISA technique

For the statistical analysis, the PASW Statistics 20 software package (SPSS Inc, Chicago, IL, USA) was used. Komolgorov-Smirnov and Levene's tests were applied to assess normal distribution and variance equality, respectively. Conventional one-way ANOVA was performed considering the experimental group as the independent variable. When SA-11 inoculation/treatment had a significant effect on the dependent variable, Scheffé's post hoc test was applied. Kruskal-Wallis and Mann-Whitney U tests were used when non-normal distribution or different variance were found. Finally, the chi-square test was used to compare diarrhea incidence. Differences were considered significant at P values of < 0·05. All the results are expressed as mean and SEM of number of animals.

### Results

### Effects on growth:

Body weight was recorded in all animals and neither the diet nor the viral infection influenced their growth. No difference was observed up to 1-week post-inoculation (day 14). This means that body weight loss due to infection or diet influence can be disregarded. However, at the end point of the experiment at day 21 (n=9/group), some differences were observed. In the RV group a significant decrease in body weight was observed compared to the REF and FM+G/F groups. If the overall body weight gain (% from day 2 to 21) is considered, this body weight loss induction in the RV group (p<0.05) and the protection by the FM+G/F intervention is still observed (see Table 1).

**Table 1: Body weight (g) during the study, before and after virus inoculation on day 7. Results are expressed as mean values and SEM (n=21 animals/group).**

| Age | BW (g) | | | | | |
|---|---|---|---|---|---|---|
| | REF | | RV | | FM+G/F | |
| Day | Mean | SEM | Mean | SEM | Mean | SEM |
| 2 | 6.45 | 0.10 | 6.92 | 0.24 | 6.22 | 0.10 |
| 7 | 11.66 | 0.24 | 11.83 | 0.22 | 11.10 | 0.20 |
| 14 | 23.32 | 0.37 | 23.17 | 0.41 | 22.96 | 0.48 |
| 21 | 34.6 | 0.66 | 28.76* | 0.42 | 36.11 | 0.40 |

| | | | | | | |
|---|---|---|---|---|---|---|
| *p < 0.05 compared to REF | | | | | | |

In the REF group a relative body weight gain of 411.11 % was observed. In the RV group this was significantly lower (p<0.05) and 310,45 %, In the FM+G/F group this reduced increase in body weight was not observed, and the relative increase was 439.90 %.

### Effect on organs

Rotavirus infection also induced some changes on organ weight on both day 14 and 21. Specifically, 7 days after infection (day 14 of life) the RV infection induced lower spleen and higher liver relative weight (p<0.05 vs. REF). The effect on the weight of the liver was also observed in the FM+G/F group, but the effect on the spleen was ameliorated in the FM+G/F group. The results from day 21 evidenced a significant impact on the RV group with lower relative weight of spleen, liver and small intestine (SI) compared to the REF group (p<0.05). Also the relative weight of the large intestine was lower. However, the supplementation with the FM+G/F mixture prevented the impact of the RV infection on those variables (p<0.05 vs RV) and the relative weight were higher and not statistically different from the reference group..

### Incidence of diarrhea

Two different approaches to evaluate the incidence of diarrhea were used: % of diarrheic animals (% DA) and % of diarrheic feces (% DF).

The % DA showed that almost all the RV-inoculated animals, 95-100%, displayed diarrhea at some time. As expected, less than 5 % of the animals from the REF group (with no RV inoculation) showed diarrhea. The % DA in the RV group was higher than 80% during the first 4 days immediately after the SA-11 inoculation (from day 8 to day 11 of life). After that, this percentage decreased, and on day 13 none of the animals in the RV group had diarrhea-like feces. The animals from the FM+G/F group displayed some improvement during the diarrhea period. Specifically, this group showed a trend towards a lowering of the % DA with respect to the RV group starting two days after the induction of the infection (day 8-11). An amelioration of the diarrhea incidence was observed, which was significantly lower than that of the RV group, on days 9 and 10 of life (p<0.05).

The calculation of diarrhea incidence as the % DF, which only takes into account the number of diarrheic feces with respect to the fecal specimens obtained, displayed the same pattern as that of the above results for all groups, and an ever higher advantageous effect was observed in the FM+G/F group; showing a lower % diarrheic feces on day 8, 9, 10, 11 and 12.

The diarrhea process in the RV group started 1-2 days after inoculation and ended around day 11 of life. Both the diarrhea period and the days with diarrhea were between 3 and 4 days. In the FM+G/F group, the length of these periods was a bit shorter, especially when looking at the % diarrheic feces (DF). In this case the duration of diarrhea was shortened and already no diarrheic feces was observed at day 11. In any case, the RV group had the maximum incidence (% DA and % DF) 3 days after the RV induction (day 10), whereas the FM+G/F group displayed the highest value the first day post-infection (day 8).

When both the % DA-AUC (Area under the curve) and the % DF-AUC were calculated, the RV group had values of 328.57 and 362.06 arbitrary units, respectively whereas REF animals did not develop diarrhea and had AUC values around 0. In the FM+G/F group a clear overall reduction was observed. The % DA-AUC was reduced to 207.14 (63 % compared to the RV group) and of % DF-AUC was reduced to 107. 87 (30 % of RV group).

### Severity of diarrhea

The Diarrhea index (DI) in the RV group was higher than 2.5 from day 8 to day 10, after which the mean score descended to approximately 2 on day 11 and even lower thereafter, indicating absence of diarrhea in most of the animals. In the FM+G/F group a significative reduction was observed and DI was below 2 in all post-infection days with exception of the first day.

The AUC of the severity pattern (sAUC) was 5.99 in the RV group. A significant reduction to 3.27 (about 55%) in sAUC was observed for the FM+G/F group (p<0.05), demonstrating an overall reduction in the severity of the process.

### Viral shedding

The viral shedding was evaluated after inoculation (days 7-16). In all RV-inoculated animals, the maximum viral elimination (p<0.054 vs REF) occurred on the first day after inoculation (day 8), whereas no differences were observed between the REF and RV groups afterwards. On the first day after inoculation, the FM+G/F intervention partially avoided such shedding increase, showing a lower viral load than the RV group (about 35 %; p<0.05) and more similar to the REF animals.

### Immune response

Serum-specific anti-RV antibodies (total, IgG, IgM and IgA) and intestinal IgA and IgM were evaluated at day 14 and 21 of life. The RV group had measurable levels of the specific anti-RV total, IgA, IgG and IgM in serum and intestine even at day 14 of life compared to the REF group.

Dietary supplementation with FM+G/F was able to modify the specific humoral immune response against the RV. On day 14, whereas the total serum anti-RV antibodies seemed not to be affected by the diet, a trend towards an increase of IgM, a significant increase of IgG (p<0.05 vs RV) and a significant decrease of IgA (p<0.05 vs RV) was observed

Regarding the intestinal compartment, there was a trend to induce higher levels of IgA on day 14 but lower levels on day 21 due to the FM+G/F diet. It seems that the mixture of FM+G/F enhances the early response (day 14) in terms of sera anti-RV IgG and intestinal anti-RV IgA

### Example 2: Rotavirus blocking experiments

An *in vitro* blocking assay was performed to test the ability of the FM+G/F, FM or G/F to bind to the SA11 rotavirus. A 96-well plate was co-incubated with 5×10⁴ particles/ml of SA-11 in PBS-Tween 1 wt.% with different dilutions of the nutrition or nutritional components (dilutions in mineral water) and incubated for 30 min. The unbound viral particles were quantified by ELISA (Perez-Cano F J, et al, 2007, Pediatr Res 2007;62: 658-663). SA-11 virus particles (10⁵ to 10³/ml) were used as standard in each plate. This assay allows the quantification of the number of SA-11 particles detected after incubation with several dilutions of the product as well as the percentage of inhibition. FM+G/F in undiluted form was 267 mg/ml, of which 211 mg/ml FM and 56 mg G/F. For FM and G/F the stock solutions were 211 mg/ml and 56 mg/ml respectively. These amounts were chosen as these correspond to the intake per day in example 1.

The results are shown in Table 2. All dilutions from the nutrition or nutritional components as used in example 1 have an inhibitory activity. In particular the FM+G/F showed a high binding activity , indicating a high blocking potential of this mixture.

**Table 2: Percentage of inhibition of RV particle detection after incubation with G/F, FM and FM+G/F at different dilutions of the nutrition and/or nutritional components. Results are expresses with their SEM of duplicates from three independent experiments.**

| Product | G/F | FM | FM+G/F |
|---|---|---|---|
| Product dilution factor) | Mean (SEM) | Mean (SEM) | Mean (SEM) |
| 1/60 | 10.87* (2.86) | 68.56* (8.66) | 91.67*# (1.94) |
| 1/6 | 19.98* (2.6) | 41.16* (8.7) | 98.12*# (0.9) |
| 1/3 | 28.44* (2.35) | 29.72* (8.2) | 98.9*# (0.37) |
| 1/2 | 39.41* (3.79) | 29.68* (10.36) | 97.16* (0.59) |

| | | | |
|---|---|---|---|
| * p < 0.05 vs % of inhibition without product addition. Nd: not determined # p < 0.05 vs % of inhibition of products with G/F or FM alone | | | |

Binding of the rotavirus particles is thought to be beneficial. Their blocking by direct interaction will avoid or diminish the entry of the rotavirus into the enterocytes and the replication in the small intestine. Binding of rotavirus particles was much higher with FM+G/F than could be expected based on the data for G/F and FM alone. These results are indicative of an unexpected improved effect of consumption of a composition comprising both a combination of partly fermented composition and non-digestible oligosaccharides on prevention and/or treatment of rotavirus infection or rotavirus induced diarrhea. In particular an effect on prevention, reduction of the viral load, of rotavirus infection can be expected, and this may result in reduced severity, reduced incidence and reduced duration of rotavirus diarrhea and/or a reduced viral shedding, when compared to compositions comprising no fermented composition or no non-digestible oligosaccharides.

### Example 3: Effect of fermented formula with non-digestible oligosaccharides on rotavirus infection in rats.

An experiment similar to example 1 was performed, except that inoculation with the RV was performed at day 5. Additional intestinal permeability was measured and caecal short chain fatty acids were measured as known in the art.

Experimental nutrition or nutritional components tested were:
1) A heat treated fermented infant formula. The fermented milk was obtained by fermentation of an infant formula with *Bifidobacterium breve* C50 and *Streptococcus thermophilus* 065 during the manufacturing process. This process added to the composition of the formula active bacterial metabolites and cell fragments (postbiotic compounds). It is available on the market in France under the name Lactofidus or Lactofidia as a product from Bledina. An additional heat step was applied to kill the lactic acid producing bacteria after fermentation step (10 min, 90 °C). The difference with the formula in example 1 is that a slightly increased fermentation and lactic-acid production takes place. The amount of L-lactic acid was about 1.1 g per 100 g.
2) A mixture of non-digestible oligosaccharides scGOS (source Vivinal^{®} GOS, Borculo Domo FrieslandCampina) and IcFOS (Source Raftiline^{®} HP, Orafti) in a 9:1 wt./wt. ratio
3) Vehicle: The vehicle of the nutritional composition or ingredients in 1 and 2 was mineral water.

Newborn, suckling G14 Lewis rats were distributed into several different experimental groups:
1) reference (REF) group, that was a control group that was not infected with rotavirus, and received no dietary intervention
2) a rotavirus (RV) group), that was a control group that was infected with rotavirus and received no dietary intervention
3) a study group (G/F), that was infected with rotavirus and that was supplemented with a mixture of non-digestible oligosaccharides scGOS/IcFOS (G/F). G/F were administered in an amount of 0.8 g/100 g body weight/day, respectively.
4) a study group (FM), that was infected with rotavirus and that was supplemented with fermented milk (FM). FM was administered in an amount of 3 g/100 g body weight/day.
5) a study group (FM+G/F), that was infected with rotavirus and that was supplemented with fermented milk (FM) as described above under and a mixture of non-digestible oligosaccharides scGOS/IcFOS (G/F). FM and G/F were administered in an amount of 3 g/100 g body weight/day and 0.8 g/100 g body weight/day, respectively.

As a control 3 groups of rats that were fed the same supplements of group 3, 4 and 5, but without a rotavirus infection, were also included. The values for diarrhea obtained in these groups were used as a baseline to normalize the diarrhea index DI and diarrhea incidence (% DA).

### Results:

Based on the diarrhea index (DI), the diarrhea episode lasted from day 6 (1 day after RV inoculation) to day 9 in the RV group 2. At day 10 the diarrhea had stopped in all groups. The duration of diarrhea as determined by the DI was the same in groups 3 and 4. The maximum DI at the diarrhea days (day 6-9) was observed in group 2. In group 5, the diarrhea, based on DI, was at day 9 already significantly lower than the RV group 2. On all days of diarrhea, the DI was lowest in group 5. This is indicative for a shorter duration of diarrhea in the experimental group 5.

The incidence of diarrhea (based on the number of animals suffering from diarrhea based on total animals, % DA) was the lowest in group 5. At day 9 only in group 5 the incidence of diarrhea was already absent, whereas it still was present in groups 2, 3 and 4, indicative for a shortened duration or rotavirus infection when the combination of FM and NDO is administered.

**Table 3: Indices of incidence of diarrhea with normalization]**

| Index | Group 2 (RV) | Group 3 (G/F) | Group 4 (FM) | Group 5 (G/F + FM) |
|---|---|---|---|---|
| MDA | 58.33 | 8.33 | 34.79 | 4.17 |
| DA-AUC | 166.66 | 29.52 | 86.23 | 8.33 |

| | | | | |
|---|---|---|---|---|
| MDA, maximum diarrhoeic animals (%), DA-AUC: Area under the curve of % DA pattern. | | | | |

The maximum diarrhea incidence (MDA), the peak incidence, was significantly lower (using a Chi-square test) in group 5 when compared with group 2. The MDA was also reduced in group 3 and 4, compared to group 2. The area under the curve of the diarrhea index in the course of the experiment was lowest in group 5 (8.33), and highest in group 2 (166.66). This value was 29.52 in group 3 and 86.23 in group 4. Again, the low value in group 5 is lower than expected based on the effects in group 3 and 4 alone. This indicative for reduced severity in the group that consumed both FM and G/F.

The intestinal permeability was determined by means of alpha-1 anti-trypsin levels in gut wash at day 8. Interestingly the combination of FM with G/F increased the gut barrier function during rotavirus infection to the largest extent.

The observed improvement in intestinal barrier function may be explained by the unexpected increase in intestinal butyric acid in group 5. Butyrate is known as a fuel for the intestinal epithelial cells and to improve the gut barrier. The level of caecal butyrate in group 2 was 239.21 µmol/g caecal material. The increased level of butyric acid in group 5 (484.39 µmol/g caecal material) is more than can be expected based on the levels found for group 3 (164.75; µmol/g caecal material, i.e. a reduction of butyrate) and 4 (309.72 µmol/g caecal material) alone.

## Claims

1. A nutritional composition that comprises a milk substrate that is fermented by lactic acid producing bacteria, and comprises non-digestible oligosaccharides selected from the group consisting of fructo-oligosaccharides, non-digestible dextrins, galacto-oligosaccharides, xylo-oligosaccharides, arabino-oligosaccharides, arabinogalacto-oligosaccharides, gluco-oligosaccharides, gentio-oligosaccharides, glucomanno-oligosaccharides, galactomanno-oligosaccharides, mannan-oligosaccharides, isomalto-oligosaccharides, nigero-oligosaccharides, glucomanno-oligosaccharides, chito-oligosaccharides, soy oligosaccharides, sialyl-oligosaccharides, and fuco-oligosaccharides, and mixtures thereof, for use in treating and/or preventing rotavirus infection or rotavirus-induced diarrhea in an infant or young child.

2. A nutritional composition that comprises a milk substrate that is fermented by lactic acid producing bacteria, and comprises non-digestible oligosaccharides selected from the group consisting of fructo-oligosaccharides, non-digestible dextrins, galacto-oligosaccharides, xylo-oligosaccharides, arabino-oligosaccharides, arabinogalacto-oligosaccharides, gluco-oligosaccharides, gentio-oligosaccharides, glucomanno-oligosaccharides, galactomanno-oligosaccharides, mannan-oligosaccharides, isomalto-oligosaccharides, nigero-oligosaccharides, glucomanno-oligosaccharides, chito-oligosaccharides, soy oligosaccharides, sialyl-oligosaccharides, and fuco-oligosaccharides, and mixtures thereof, for use in
a. reducing incidence of rotavirus-induced diarrhea or rotavirus infection,
b. reducing duration of rotavirus-induced diarrhea or rotavirus infection,
c. reducing severity of rotavirus-induced diarrhea or rotavirus infection,
d. preventing of rotavirus-induced reduced growth or poor growth and/or reduced development or poor development,
e. increasing blocking of rotavirus particles,
f. reducing rotaviral load, and/or
g. increasing gut barrier function during rotavirus infection,
in an infant or young child.

3. The nutritional composition for use according to claim 2, wherein the reduction, prevention or increase is observed when compared to an infant or young child being administered a nutritional composition that is not at least partly fermented and does not comprise non-digestible oligosaccharides.

4. The nutritional composition for use according to any one of the preceding claims, wherein the nutritional composition comprises 0.1 to 1.5 wt.% of the sum of lactic acid and lactate based on dry weight of the composition, and preferably wherein the sum of L-lactic acid and L-lactate is more than 50 wt.% based on the sum of total lactic acid and lactate.

5. The nutritional composition for use according to any one of the preceding claims, wherein the nutritional composition comprises lactic acid producing bacteria, preferably selected from the group consisting of Bifidobacterium and Streptococcus, preferably both.

6. The nutritional composition for use according to any one of claims 1-4, wherein the nutritional composition comprises inactivated lactic acid producing bacteria.

7. The nutritional composition for use according to any one of the preceding claims wherein the nutritional composition comprises 2.5 to 15 wt.% non-digestible oligosaccharides based on dry weight of the nutritional composition.

8. The nutritional composition for use according to any one of the preceding claims wherein the non-digestible oligosaccharides are selected from the group consisting of galacto-oligosaccharides and fructo-oligosaccharides, preferably both.

9. The nutritional composition for use according to any one of the preceding claims wherein the nutritional composition does not comprise pectin-derived acidic oligosaccharides, in particular galacturonic acid oligosaccharides.

10. The nutritional composition for use according to any one of the preceding claims wherein the nutritional composition is an infant formula or a follow-on formula.

## Patentansprüche

1. Nährstoffzusammensetzung, die ein Milchsubstrat umfasst, das durch Milchsäure erzeugende Bakterien fermentiert wird, und die nicht verdauliche Oligosaccharide umfasst, die aus der Gruppe ausgewählt sind, die aus Fructo-Oligosacchariden, nicht verdaulichen Dextrinen, Galacto-Oligosacchariden, Xylo-Oligosacchariden, Arabino-Oligosacchariden, Arabinogalacto-Oligosacchariden, Gluco-Oligosacchariden, Gentio-Oligosacchariden, Glucomanno-Oligosacchariden, Galactomanno-Oligosacchariden, Mannan-Oligosacchariden, Isomalto-Oligosacchariden, Nigero-Oligosacchariden, Glucomanno-Oligosacchariden, Chito-Oligosacchariden, Soja-Oligosacchariden, Sialyl-Oligosacchariden und Fuco-Oligosacchariden und Mischungen dieser besteht, zur Anwendung bei der Behandlung und/oder Vorbeugung von Rotavirus-Infektionen oder Rotavirus-induzierter Diarrhöe bei einem Säugling oder jungen Kind.

2. Nährstoffzusammensetzung, die ein Milchsubstrat umfasst, das durch Milchsäure erzeugende Bakterien fermentiert wird, und die nicht verdauliche Oligosaccharide umfasst, die aus der Gruppe ausgewählt sind, die aus Fructo-Oligosacchariden, nicht verdaulichen Dextrinen, Galacto-Oligosacchariden, Xylo-Oligosacchariden, Arabino-Oligosacchariden, Arabinogalacto-Oligosacchariden, Gluco-Oligosacchariden, Gentio-Oligosacchariden, Glucomanno-Oligosacchariden, Galactomanno-Oligosacchariden, Mannan-Oligosacchariden, Isomalto-Oligosacchariden, Nigero-Oligosacchariden, Glucomanno-Oligosacchariden, Chito-Oligosacchariden, Soja-Oligosacchariden, Sialyl-Oligosacchariden und Fuco-Oligosacchariden und Mischungen dieser besteht, zur Anwendung bei der
a. Verringerung des Auftretens von Rotavirus-induzierter Diarrhöe oder einer Rotavirus-Infektion,
b. Verkürzung der Dauer der Rotavirus-induzierten Diarrhöe oder Rotavirus-Infektion,
c. Verringerung des Schweregrads der Rotavirus-induzierten Diarrhöe oder Rotavirus-Infektion,
d. Verhinderung eines Rotavirus-induzierten verminderten Wachstums oder schlechten Wachstums und/oder einer verminderten Entwicklung oder schlechten Entwicklung,
e. Erhöhung der Blockierung von Rotavirus-Partikeln,
f. Verringerung der Rotavirus-Belastung, und/oder
g. Erhöhung der Darmbarrierefunktion während einer Rotavirus-Infektion,
bei einem Säugling oder jungen Kind.

3. Nährstoffzusammensetzung zur Anwendung nach Anspruch 2, wobei die Verringerung, Verhinderung oder Erhöhung im Vergleich zu einem Säugling oder jungen Kind beobachtet wird, dem eine Nährstoffzusammensetzung verabreicht wird, die nicht zumindest teilweise fermentiert ist und keine unverdaulichen Oligosaccharide enthält.

4. Nährstoffzusammensetzung zur Anwendung nach einem der vorangehenden Ansprüche, wobei die Nährstoffzusammensetzung 0,1 bis 1,5 Gew.-% der Summe von Milchsäure und Lactat, bezogen auf das Trockengewicht der Zusammensetzung, umfasst, und wobei die Summe von L-Milchsäure und L-Lactat vorzugsweise mehr als 50 Gew.-%, bezogen auf die Summe von Gesamtmilchsäure und Lactat, beträgt.

5. Nährstoffzusammensetzung zur Anwendung nach einem der vorangehenden Ansprüche, wobei die Nährstoffzusammensetzung Milchsäure produzierende Bakterien umfasst, vorzugsweise ausgewählt aus der Gruppe bestehend aus Bifidobacterium und Streptococcus, vorzugsweise beide.

6. Nährstoffzusammensetzung zur Anwendung nach einem der Ansprüche 1 - 4, wobei die Nährstoffzusammensetzung inaktivierte Milchsäure produzierende Bakterien umfasst.

7. Nährstoffzusammensetzung zur Anwendung nach einem der vorangehenden Ansprüche, wobei die Nährstoffzusammensetzung 2,5 bis 15 Gew.-% nicht verdauliche Oligosaccharide, bezogen auf das Trockengewicht der Nährstoffzusammensetzung, umfasst.

8. Nährstoffzusammensetzung zur Anwendung nach einem der vorangehenden Ansprüche, wobei die unverdaulichen Oligosaccharide aus der Gruppe ausgewählt sind, die aus Galacto-Oligosacchariden und Fructo-Oligosacchariden, vorzugsweise beiden, besteht.

9. Nährstoffzusammensetzung zur Anwendung nach einem der vorangehenden Ansprüche, wobei die Nährstoffzusammensetzung keine von Pektin abgeleiteten sauren Oligosaccharide, insbesondere Galakturonsäure-Oligosaccharide, enthält.

10. Nährstoffzusammensetzung zur Anwendung nach einem der vorangehenden Ansprüche, wobei die Nährstoffzusammensetzung eine Säuglingsanfangsnahrung oder eine Folgenahrung ist.

## Revendications

1. Composition nutritionnelle qui comprend un substrat de lait qui est fermenté par des bactéries produisant de l'acide lactique, et qui comprend des oligosaccharides non digestibles sélectionnés dans le groupe constitué par des fructo-oligosaccharides, des dextrines non digestibles, des galacto-oligosaccharides, des xylo-oligosaccharides, des arabino-oligosaccharides, des arabinogalacto-oligosaccharides, des gluco-oligosaccharides, des gentio-oligosaccharides, des glucomanno-oligosaccharides, des galactomanno-oligosaccharides, des mannan-oligosaccharides, des isomalto-oligosaccharides, des nigéro-oligosaccharides, des glucomanno-oligosaccharides, des chito-oligosaccharides, des oligosaccharides de soja, des sialyl-oligosaccharides, et des fuco-oligosaccharides, et des mélanges de ceux-ci, à utiliser pour traiter et/ou prévenir une infection à rotavirus ou une diarrhée induite par rotavirus chez un nourrisson ou un jeune enfant.

2. Composition nutritionnelle qui comprend un substrat de lait qui est fermenté par des bactéries produisant de l'acide lactique, et qui comprend des oligosaccharides non digestibles sélectionnés dans le groupe constitué par des fructo-oligosaccharides, des dextrines non digestibles, des galacto-oligosaccharides, des xylo-oligosaccharides, des arabino-oligosaccharides, des arabinogalacto-oligosaccharides, des gluco-oligosaccharides, des gentio-oligosaccharides, des glucomanno-oligosaccharides, des galactomanno-oligosaccharides, des mannan-oligosaccharides, des isomalto-oligosaccharides, des nigéro-oligosaccharides, des glucomanno-oligosaccharides, des chito-oligosaccharides, des oligosaccharides de soja, des sialyl-oligosaccharides et des fuco- oligosaccharides, et des mélanges de ceux-ci, à utiliser dans les étapes consistant à
a. réduire une incidence de diarrhée induite par rotavirus ou d'infection à rotavirus,
b. réduire la durée de la diarrhée induite par rotavirus ou de l'infection à rotavirus,
c. réduire la gravité de la diarrhée induite par rotavirus ou de l'infection à rotavirus,
d. prévenir une croissance réduite ou d'une faible croissance induite par rotavirus et/ou un développement réduit ou un faible développement,
e. augmenter le blocage de particules de rotavirus,
f. réduire la charge rotavirale, et/ou
g. augmenter la fonction de barrière intestinale pendant l'infection à rotavirus, chez un nourrisson ou un jeune enfant.

3. Composition nutritionnelle à utiliser selon la revendication 2, dans laquelle la réduction, la prévention ou l'augmentation est observée par comparaison à un nourrisson ou à un jeune enfant auquel est administrée une composition nutritionnelle qui n'est pas au moins partiellement fermentée et qui ne comprend pas d'oligosaccharides non digestibles.

4. Composition nutritionnelle à utiliser selon l'une quelconque des revendications précédentes, dans laquelle la composition nutritionnelle comprend de 0,1 à 1,5 % en poids de la somme d'acide lactique et de lactate sur la base du poids sec de la composition, et de préférence dans laquelle la somme d'acide L-lactique et de L-lactate est supérieure à 50 % en poids sur la base de la somme d'acide lactique total et de lactate.

5. Composition nutritionnelle à utiliser selon l'une quelconque des revendications précédentes, dans laquelle la composition nutritionnelle comprend des bactéries produisant de l'acide lactique, de préférence sélectionnées dans le groupe constitué de Bifidobacterium et de Streptococcus, de préférence les deux.

6. Composition nutritionnelle à utiliser selon l'une quelconque des revendications 1 à 4, dans laquelle la composition nutritionnelle comprend des bactéries produisant de l'acide lactique inactivées.

7. Composition nutritionnelle à utiliser selon l'une quelconque des revendications précédentes, dans laquelle la composition nutritionnelle comprend de 2,5 à 15 % en poids d'oligosaccharides non digestibles sur la base du poids sec de la composition nutritionnelle.

8. Composition nutritionnelle à utiliser selon l'une quelconque des revendications précédentes, dans laquelle les oligosaccharides non digestibles sont sélectionnés dans le groupe constitué de galacto-oligosaccharides et de fructo-oligosaccharides, de préférence les deux.

9. Composition nutritionnelle à utiliser selon l'une quelconque des revendications précédentes, dans laquelle la composition nutritionnelle ne comprend pas d'oligosaccharides acides dérivés de la pectine, en particulier d'oligosaccharides d'acide galacturonique.

10. Composition nutritionnelle à utiliser selon l'une quelconque des revendications précédentes, dans laquelle la composition nutritionnelle est une préparation pour nourrisson ou une préparation de suite.
